# EUROPEAN PATENT SPECIFICATION

(11) **EP 4 048 354 B1**
(45) Date of publication and mention of the grant of the patent: **04.10.2023**
(21) Application number: 20797203.5
(22) Date of filing: 23.10.2020
(51) Int. Cl.: A24F 42/20, A61M 11/00, A61M 15/00, A61M 15/06

(54) **INHALER ARTICLE WITH TANGENTIAL AIRFLOW END ELEMENT**
INHALATORARTIKEL MIT TANGENTIALEM LUFTSTROMENDELEMENT
ARTICLE INHALATEUR DOTÉ D'UN ÉLÉMENT D'EXTRÉMITÉ DE FLUX D'AIR TANGENTIEL

(30) Priority: 25.10.2019 EP 19205460
(43) Date of publication of application: 31.08.2022
(73) Proprietor: Philip Morris Products S.A., 2000 Neuchâtel (CH)
(72) Inventor: CAMPITELLI, Gennaro, 2000 Neuchâtel (CH)
(74) Representative: Parsi Mendiola, Joshua
(86) International application number: PCT/IB2020/059993
(87) International publication number: WO 2021/079344

(56) References cited:
- WO-A1-2017/109626
- WO-A1-2018/100461
- WO-A1-2019/082057
- WO-A1-2019/186337
- GB-A- 1 396 258

## Description

This disclosure relates to an inhaler article having a tangential airflow end element and inhaler systems including the inhaler article.

WO 2019/186337 A1 describes an inhaler article including a body extending from a mouthpiece end to a distal end with an endpiece element at the distal end. A capsule cavity is defined within the body and extends a cavity length. An air inlet region is between the endpiece element and the capsule cavity. The air inlet region has an air inlet and an air passageway extending from the air inlet to the capsule cavity. The air passageway has an inner diameter less than an inner diameter of the capsule cavity.

Dry powder inhalers are not always fully suitable to provide dry powder particles to the lungs at inhalation or air flow rates that are within conventional smoking regime inhalation or air flow rates. Dry powder inhalers may be complex to operate or may involve moving parts. Dry powder inhalers often strive to provide an entire dry powder dose or capsule load in a single breath.

Dry powder inhalers are typically formed of materials that are not biodegradable. In addition, these dry powder inhalers are often formed of materials that may need to be injection moulded or cast and assembly of these parts may present a bottleneck in the manufacturing process and are difficult to produce at high speeds.

It would be desirable to provide a dry powder inhaler that is substantially biodegradable. It would be desirable to provide an inhaler article that may be assembled at high speeds. It would also be desirable to provide an inhaler article that has a form that is easy to hold and is familiar to a user, similar to a conventional cigarette. It would also be desirable to provide an inhaler article that is convenient to use by a consumer.

According to an aspect of the present invention, there is provided an inhaler article including a body extending along a longitudinal axis from a mouthpiece end to a distal end, a capsule cavity defined within the body and a tubular element extending from the distal end towards the capsule cavity. The tubular element defines a central passage having uniform open diameter extending from the distal end towards the capsule cavity. The tubular element includes at least one tangential air inlet allowing air to enter into the central passage. The at least one tangential air inlet extends in a direction that is tangential to the central passage.

Advantageously, the inhaler article provides a simplified construction that utilizes tangential inhalation airflow into the central passage to induce rotational or swirling airflow within the tubular element. This rotational or swirling airflow is transmitted to a capsule cavity of an inhaler article. The rotational or swirling airflow induces a capsule contained within the capsule cavity to rotate and release particles into the rotational or swirling airflow to the consumer.

Preferably the at least one tangential air inlet is proximate to the distal end of the inhaler article body. The central passage may include a first end defining an upstream boundary of the capsule cavity and a second opposing end defining the distal end of the inhaler article body. Preferably, the second opposing end defines an open distal end of the of the inhaler article body. The central passage may extend along the longitudinal axis of the body of the inhaler article and define an opening at the distal end of the body of the inhaler article that is coaxial with the longitudinal axis of the body of the inhaler article. Preferably, a lateral distance between the at least one air inlet and the capsule cavity is greater than a lateral distance from the distal end of the inhaler article body to the at least one air inlet.

Advantageously, the tubular element may define an open aperture along the longitudinal axis and may not have an element blocking or occluding the open distal end of the inhaler article, to reduce complexity of the inhaler article. The consumer may simply occlude or block the open distal end with a holder or the consumer's finger to direct inhalation airflow substantially through the tangential air inlets on the inhaler article.

The tubular element may comprise at least two air inlets. The at least two air inlets extend in a direction that is tangential to the central passage. The open tubular element may comprise at least three air inlets. The at least three air inlets extend in a direction that is tangential to the central passage.

Preferably the tubular element is formed of a biodegradable material. Preferably the tubular element comprises a fibrous material. Preferably the tubular element is formed of a porous material. Preferably the tubular element is formed of a cellulose material, such as cellulose acetate. Preferably, the tubular element is formed of a polylactic acid material.

Advantageously, the tubular element may be formed of materials used to assemble conventional cigarettes. Advantageously, the inhaler article may be formed of biodegradable materials.

Preferably, a capsule is retained within the capsule cavity. The tubular element central passage may have a diameter in a range from about 50% to about 90% of a diameter of the capsule. It should be appreciated that as the diameter of the tubular element central passage increases the overall diameter of the tubular element may increase also in order to provide air inlets of sufficient length in order to guide the airflow into a tangential airflow.

Advantageously, the distal end tubular element may produce a "swirling" airflow from the tangential air inlet into the central passage of the tubular element. This swirling airflow is useful for effective depletion of the capsule during consumption, after the capsule has been pierced. Advantageously, "swirling" effect may cause rotation of the capsule to provide a uniform entrainment of a portion or a fraction of nicotine particles from the capsule over two or more, or five or more, or ten or more inhalations or "puffs" by a user.

According to an aspect of the present technology, there is provided an inhaler system including, the inhaler article described herein with a capsule disposed within the capsule cavity and a holder receiving the inhaler article. The holder includes a housing having a housing cavity for receiving an inhaler article and a sleeve configured to retain an inhaler article within the housing cavity. The sleeve includes a sleeve cavity and the sleeve is movable within the housing cavity along a longitudinal axis of the housing. The sleeve includes a first open end and a second opposing end. The first open end is configured to receive an inhaler article and the second opposing end of the sleeve is configured to contact the distal end of the inhaler article.

Preferably the sleeve second opposing end is configured to direct substantially all inhalation air to flow through the the inhaler article at least one air inlet extending in a direction that is tangential to the central passage.

Advantageously, the holder may cooperate with the inhaler article to direct substantially all of the inhalation air flow through the tangential air inlets on the inhaler article.

Preferably the holder further includes a piercing element fixed to and extending from a housing inner surface. The piercing element is configured to extend through the second opposing end of the sleeve and into the capsule cavity to pierce the capsule along a longitudinal axis of the housing.

Preferably, the capsule contains pharmaceutically active particles. The pharmaceutically active particles may comprise nicotine. The pharmaceutically active particles may have a mass median aerodynamic diameter of about 5 micrometres or less, or in a range from about 0.5 micrometres to about 4 micrometres, or in a range from about 1 micrometres to about 3 micrometres.

The inhaler article described herein may be combined with a piercing element or holder including a piercing element to deliver the nicotine particles from the capsule to a user. The piercing element or piercing device (or holder) may be separated from or not form a portion of the inhaler article. A plurality of the inhaler articles may be combined with a piercing element or piercing device (or holder) to form a kit.

Advantageously, the inhaler system efficiently provides nicotine particles to the lungs at inhalation or air flow rates that are within conventional smoking regime inhalation or air flow rates. The inhaler delivers the nicotine powder with an inhaler article that has a form similar to a conventional cigarette. The inhaler system described herein may provide a dry powder to the lungs at inhalation or air flow rates that are within conventional smoking regime inhalation or air flow rates. A consumer may take a plurality of inhalations or "puffs" where each "puff" delivers a fractional amount of dry powder contained within a capsule contained within the capsule cavity. This inhaler article may have a form similar to a conventional cigarette and may mimic the ritual of conventional smoking. This inhaler article may be simple to manufacture and convenient to use by a consumer.

Air flow management through a capsule cavity of the inhaler article may cause a capsule contained therein to rotate during inhalation and consumption. The capsule may contain particles containing nicotine (also referred to as "nicotine powder" or "nicotine particles") and optionally particles comprising flavour (also referred to as "flavour particles"). Rotation of the pierced capsule may suspend and aerosolize the nicotine particles released from the pierced capsule into the inhalation air moving through the inhaler article. The flavour particles may be larger than the nicotine particles and may assist in transporting the nicotine particles into the lungs of the user while the flavour particles preferentially remain in the mouth or buccal cavity of the user. The nicotine particles and optional flavor particles may be delivered with the inhaler article at inhalation or air flow rates that are within conventional smoking regime inhalation or air flow rates.

The term "nicotine" refers to nicotine and nicotine derivatives such as free-base nicotine, nicotine salts and the like.

The term "flavourant" or "flavour" refers to organoleptic compounds, compositions, or materials that alter and are intended to alter the taste or aroma characteristics of nicotine during consumption or inhalation thereof.

The terms "upstream" and "downstream" refer to relative positions of elements of the holder, inhaler article and inhaler systems described in relation to the direction of inhalation air flow as it is drawn through the body of the holder, inhaler article and inhaler systems.

The terms "proximal" and "distal" are used to describe the relative positions of components, or portions of components, of the inhaler article, holder, or system. Holders or elements (such as the sleeve) forming the holder, according to the technology have a proximal end which, in use, receives an inhaler article and an opposing distal end which may be a closed end, or have an end closer to the proximal end of the holder. Inhaler articles, according to the technology have a proximal end. In use, the nicotine particles exit the proximal end of the inhaler article for delivery to a user. The inhaler has a distal end opposing the proximal end. The proximal end of the inhaler article may also be referred to as the mouth end.

The holder for an inhaler article may be combined with an inhaler article (described herein) containing a capsule for activating the inhaler article by piercing the capsule, providing reliable activation of the capsule (by puncturing the capsule with the piercing element of the holder) within inhaler article, and releasing the particles contained inside the capsule and enabling the article to deliver the particles to a consumer. The holder is separate from the inhaler article, but the consumer may utilize both the inhaler article and the holder while consuming the particles released within the inhaler article. A plurality of these inhaler articles may be combined with a holder to form a system or kit. A single holder may be utilized on 10 or more, or 25 or more, or 50 or more, or 100 or more, inhaler articles to activate (puncture or pierce) a capsule contained within each inhaler article and provide reliable activation and optionally, a visual indication (marking), for each inhaler article of the activation of the inhaler article.

A holder for an inhaler article includes a housing comprising a housing cavity for receiving an inhaler article and a sleeve configured to retain an inhaler article within the housing cavity. The sleeve comprising a sleeve cavity and being movable within the housing cavity along the longitudinal axis of the housing. The sleeve comprises a first open end and a second opposing end. The first open end is configured to receive the distal end of the inhaler article. The second opposing end of the sleeve is configured to contact the distal end of the inhaler article. The sleeve second opposing end is configured to direct substantially all inhalation air to flow through the the inhaler article at least one air inlet extending in a direction that is tangential to the central passage.

An inhaler system includes the inhaler article described herein with a capsule disposed within the capsule cavity and a holder receiving the inhaler article. The holder includes a housing having a housing cavity for receiving an inhaler article and a sleeve configured to retain an inhaler article within the housing cavity. The sleeve includes a sleeve cavity and the sleeve is movable within the housing cavity along a longitudinal axis of the housing. The sleeve includes a first open end and a second opposing end. The first open end is configured to receive an inhaler article and the second opposing end of the sleeve is configured contact the distal end of the inhaler article.

A method includes, inserting an inhaler article into the sleeve of the holder for an inhaler article, as described herein, until the distal end of the inhaler article contacts the second opposing end of the sleeve. The inhaler article includes a body, the body extending along an inhaler longitudinal axis from a mouthpiece end to a distal end, a body length, and a capsule disposed within the inhaler article body. Then, moving the inhaler article and sleeve toward the piercing element until the piercing element pierces the capsule. Then drawing air into the second opposing end of the sleeve of the holder to direct inhalation airflow into the tangential air inlets on the inhaler article to form rotational or swirling airflow through the central passage of the tubular element of the inhaler article. This swirling inhalation airflow is transmitted into the capsule cavity while the inhaler article is disposed within the holder for an inhaler article. The consumed inhaler article may then be removed from the holder and discarded. Then a fresh inhaler article may be inserted into the holder and the method repeated.

An inhaler article includes a body extending along a longitudinal axis from a mouthpiece end to a distal end, a capsule cavity defined within the body and a tubular element disposed at the distal end and extending from the distal end to the capsule cavity. The tubular element defines a central passage extending from the distal end to the capsule cavity. The tubular element includes at least one air inlet allowing air to enter into the central passage. The at least one air inlet extends in a direction that is tangential to the central passage.

The body of the inhaler article, or the "inhaler body", may have any suitable shape. The body of the inhaler article, or "inhaler body" may resemble a smoking article or conventional cigarette in size and shape. The inhaler body may have an elongated cylindrical body extending along the longitudinal axis of the inhaler article. In other words, the inhaler body may have a length that is substantially greater than the other dimensions of the inhaler body. The inhaler body may have a substantially uniform outer diameter along the length of the inhaler body. The inhaler body may have a substantially uniform inner diameter along the length of the inhaler body. The inhaler body may have any suitable transverse cross-sectional shape. For example, the transverse cross-section may be circular, elliptical, square or rectangular. The inhaler body preferably has a circular cross-section that may be uniform along the length of the inhaler body, forming an elongated cylindrical body.

The inhaler body may have an outer diameter in a range from about 6 mm to about 10 mm, or from about 7 mm to about 10 mm, or about 7 mm to about 9 mm, or about 7 mm to about 8 mm or about 7.2 mm. The inhaler body may have a length (along the longitudinal axis) in a range from about 40 mm to about 100 mm, or from about 40 mm to about 80 mm, or about 40 mm to about 65 mm. Preferably, the length of the inhaler body is the same as the length of the inhaler article. Preferably, the length of the inhaler article is about 45 mm. Preferably, the length of the inhaler body is selected such that the mouthpiece end of the inhaler article protrudes from the holder.

The inhaler body may be formed of a polymeric or cellulosic material, or any other suitable material. The inhaler body may be formed of a biodegradable material. The inhaler body may be formed of paperboard or cardboard. The inhaler body may have a uniform thickness along its length. The inhaler body may have a thickness in a range from about 1 mm to about 2 mm.

The inhaler body may form a unitary construction where the body extends continuously from the tubular element to the mouthpiece end. The distal end tubular element, the capsule cavity (and capsule if present), and a porous support element (or filter) may be serially disposed within the inhaler body. In other words, the distal end tubular element, the capsule cavity (and capsule if present), the porous support element (or filter) may be arranged end to end along the longitudinal axis of the inhaler body. The porous support element (or filter) may be disposed in a mouthpiece of the body. The mouthpiece air channel may extend from the capsule cavity to the mouthpiece end. The porous support element (or filter) may extend from the capsule cavity to the mouthpiece end of the inhaler article.

The inhaler body may be formed of two or more portions. The two or more portions may be axially aligned in serial abutting relationship and joined together to form the inhaler body. A wrapper may be utilized to join the two or more portions together. The wrapper may be a biodegradable material. The wrapper may be a paper wrapper.

The distal end tubular element is configured to induce a swirling air flow to the capsule cavity of the inhaler body. The distal end tubular element may include an open central passage with tangential air inlets for inducing swirling inhalation airflow to the capsule cavity. The distal end tubular element may induce rotational air flow or swirling air flow as the air flows through the tangential air inlets and through the capsule cavity.

Air flow through the inhaler article preferably enters the inhaler article through the inhaler article sidewall and into the central passage and then along the longitudinal axis of the inhaler article, via the capsule and filter, to exit at the mouthpiece end at the proximal end of the inhaler article.

The tubular element may be disposed at the distal end of the body. The tubular element may define the distal end of the inhaler article.

The tubular element defines an open central passage ("central passage"). The central passage may include a first end defining an upstream boundary of the capsule cavity and a second opposing end defining the distal end of the inhaler article body.

The second opposing end may define an open distal end of the of the inhaler article body. The central passage may extend along the longitudinal axis of the body of the inhaler article and define an opening at the distal end of the body of the inhaler article that is coaxial with the longitudinal axis of the body of the inhaler article. The central passage may define an open aperture extending longitudinally from the distal end of the inhaler article to the capsule cavity. During use the consumer may block or occlude the distal end open aperture so that substantially all inhalation air flow through the tangential air inlets (through the tubular element sidewall) to the central passage and be directed through the capsule cavity and out the mouth end of the inhaler article.

Blocking or occluding the second opposing end of the tubular element allows the tubular element to define a swirl generating element that is configured to generate swirling or rotational inhalation airflow. This swirling or rotational inhalation airflow may be transmitted into capsule cavity to rotate a capsule and release dry powder contained within the capsule.

The tubular element has one or more air inlets proximate to or in the vicinity of the distal end of the inhaler article. The one or more air inlets allow inhalation air to enter the central passage of the tubular element. The at least one air inlet extends in a direction that is tangential to the central passage.

The tubular element has two or more air inlets proximate to or in the vicinity of the distal end of the inhaler article. The two or more air inlets allow inhalation air to enter the central passage of the tubular element. The at least two air inlets extend in a direction that is tangential to the central passage.

The tubular element has three or more air inlets proximate to or in the vicinity of the distal end of the inhaler article. The three or more air inlets allow inhalation air to enter the central passage of the tubular element. The at least three air inlets extend in a direction that is tangential to the central passage.

The tubular element four air inlets proximate to or in the vicinity of the distal end of the inhaler article. The four air inlets allow inhalation air to enter the central passage of the tubular element. The four air inlets extend in a direction that is tangential to the central passage.

The tubular element may be coaxial with the longitudinal axis of the inhaler article.

The one or more air inlets may extend through the sidewall forming the tubular element. Preferably the one or more air inlets enter the central passage in a direction tangential to the inner surface defining the central passage.

The at least one air inlet that extends in a direction that is tangential to the central passage enters the central passage proximate to the second opposing end of the tubular member. The at least one air inlet that extends in a direction that is tangential to the central passage enters the central passage proximate to the open distal end of the inhaler article. Preferably the at least one air inlet that extends in a direction that is tangential to the central passage enters the central passage at the second opposing end of the tubular member. Preferably the at least one air inlet that extends in a direction that is tangential to the central passage enters the central passage at the distal end of the inhaler article. Improved capsule depletion occurs when the tangential air inlets are located closer to the second opposing end of the tubular member.

Preferably, a lateral distance between the at least one air inlet and the capsule cavity is greater than a lateral distance from the distal end of the inhaler article body to the at least one air inlet.

The tubular element may have a length between the first end and the second opposing end that extends along the longitudinal axis of the inhaler body. The tubular element may have any suitable length, such as between about 3 mm to about 10 mm. The tubular element may have a length of in the range of about 5 mm to about 8 mm. The central passage length may be equal to the length of the tubular element.

The tubular element may substantially fill the inner diameter of the inhaler body. The tubular element may have an outer diameter in a range from about 6 mm to about 8 mm. The tubular element may be sized and shaped to fit within and against the inner surface of the body. The tubular element may have an outer diameter sufficient to form a friction fit or interference fit with the inner surface of the inhaler body.

The open diameter of the central passage may be in a range from about 2 mm to about 5 mm, or about 3 mm to about 4 mm.

The at least one air inlet is proximate to the distal end of the inhaler article. Placement of the least one air inlet near, close to, or proximate the second opposing end of the tubular element may improve the depletion of the particles from the capsule as compared to placing the least one air inlet closer to the first end of the tubular element.

Preferably the least one air inlet is placed within about 2 mm or within about 1 mm from the distal end of the inhaler article. Preferably the least one air inlet is placed within about 2 mm or within about 1 mm from the second opposing end of the tubular element. Preferably the least one air inlet is placed within about 2 mm or within about 1 mm from both the second opposing end of the tubular element and the distal end of the inhaler article.

The tubular element may be formed of a fibrous material. The tubular element may be formed of a porous material. The tubular element may be formed of a biodegradable material. The tubular element may be formed of a cellulose material, such as cellulose acetate. The tubular element may be formed of a polylactic acid material.

The central passage has a uniform inner or open diameter extending from the capsule cavity to the open distal end or upstream-most end of the inhaler article. The central passage may have an inner diameter that is at least about 50%, or at least about 70%, or at least about 75% of a diameter of the body distal end. The central passage may have an inner diameter that is in a range from about 50% to about 90% of a diameter of the capsule retained within the capsule cavity.

The central passage may have a uniform inner or open diameter in a range from about 3 mm about 6.5 mm, or from about 4 mm to about 6 mm, or from about 5 mm to about 6 mm or about 5.5 mm.

The open tubular element defining an open central passage may have a thickness in a range from about 0.5 mm to about 1.5 mm, or about 0.5 mm to about 1 mm.

The filter element located downstream of the capsule cavity may extend from the capsule cavity to the mouthpiece end of the inhaler article. The filter element may have a length in a range from about 10 mm to about 30 mm, preferably from about 15 mm to about 25 mm and more preferably from about 20 mm to about 22 mm.

The capsule cavity may be axially aligned and in downstream serial arrangement with the tubular element. The tubular element may form an upstream or distal boundary of the capsule cavity. The capsule cavity may define a cylindrical space configured to contain a capsule. The capsule cavity may define a space configured to receive a capsule having an obround or rounded rectangular shape. The capsule cavity may have a substantially uniform or uniform diameter along the length of the capsule cavity. The capsule cavity may have a circular transverse cross-section along the length of the capsule cavity. The capsule cavity may have a cylindrical shape. The configuration of the capsule cavity relative to the capsule may allow the capsule to rotate with some stability within the capsule cavity. The longitudinal axis of the capsule may rotate with some stability about the longitudinal axis of the inhaler body during inhalation.

The capsule cavity may have a fixed cavity length bounded on an upstream end of the first end of the tubular element and bounded on the downstream end by a porous support element or filter. The capsule cavity may have a cavity length of about at least about 110% to less than about 200% of a length of the capsule contained therein, or from about 120% to about 130% of the capsule length, or about 125% of the capsule length. The cavity length may be in a range from about 15 mm to about 25 mm and the capsule length may be in a range from about 14 to about 18 mm, or the cavity length may be about 20 mm and the capsule length may be about 16 mm. The cavity length may be about 20 mm. The capsule cavity and the tubular element may have a combined length from about 25 mm to about 35 mm, or the combined length may be about 27 mm to about 32 mm.

The capsule cavity has a cavity inner diameter, orthogonal to the longitudinal axis, and the capsule has a capsule outer diameter. The capsule outer diameter may be in a range from about 80% to about 99% of the cavity inner diameter, or capsule outer diameter may be in a range from about 85% to about 95% of the cavity inner diameter, or capsule outer diameter may be about 90% of the cavity inner diameter. The capsule outer diameter may be in a range from about 5.4 mm to about 6.4 mm and the cavity inner diameter may be in a range from about 6 mm to about 7 mm.

An insert body may be included within the capsule cavity. The insert body may define a cylindrical space configured to contain a capsule. The insert body may provide additional rigidity to the body along the capsule cavity. The insert body may have a substantially uniform or uniform diameter along the length of the capsule cavity. The insert body may have a length of about at least about 110% to less than about 200% of a length of the capsule contained therein, or from about 120% to about 130% of the capsule length, or about 125% of the capsule length. The insert body may be formed of the same or different material as the body. Preferably the insert body is formed of paperboard or cardboard.

The capsule cavity may be bounded on an upstream end by the by the tubular element and bounded on a downstream end or mouthpiece side by the porous support element or filter. These elements may be joined in abutting axial alignment with a wrapper.

The tubular element and porous support element or filter may cooperate to contain the capsule longitudinally within the capsule cavity. The tubular element and the porous support element may each fill the inner diameter of the elongated inhaler body. The porous support element may allow air flow to exhibit a uniform airflow along the cross-section of the elongated inhaler body through the porous support element. The porous support element may function as a filter or diffuser to reduce turbulence effects or edge effects and ensure or maintain the desired air flow pattern through the capsule cavity. The porous support element may support a capsule inside the capsule cavity during activation of the capsule, such as by providing a support for the capsule as a piercing element is received in the inhaler article at the distal end and pierces the capsule to activate the capsule.

A capsule may be sealed within the inhaler article prior to consumption. For transport and storage, the inhaler article may be contained within a sealed or airtight container or bag. The inhaler article may include one or more peelable seal layers to cover the one or more air inlet channels at the distal end or the air outlet at the mouthpiece end of the inhaler article. This may ensure the inhaler articles maintain appropriate hygiene and freshness or may prevent the capsule from drying out and becoming hard or friable.

The capsule may rotate about its longitudinal or central axis when air is drawn through the inhaler article. The capsule may be formed of an airtight material that substantially contains the particles inside the capsule. The capsule may be configured to be pierced or punctured by a piercing element when the capsule is within the capsule cavity. The piercing element may be separate or combined with the inhaler article. The capsule may be formed of any suitable material. The capsule may be formed of a metallic or polymeric material that serves to keep contaminants out of the capsule but may be pierced or punctured by a piercing element prior to consumption to enable the release of the nicotine particles from within the capsule. The capsule may be formed of a polymer material. The polymer material may be hydroxypropylmethylcellulose (HPMC). The capsule may be any suitable size. The capsule may be a size 1 to size 4 capsule, or a size 3 capsule, or a size 3 capsule.

The system may comprise a separate piercing element, such as a metal or rigid needle. The piercing element may form a single aperture through the capsule received in the capsule cavity. The piercing element may be configured to pass through the tubular element and into the capsule cavity.

The capsule may contain pharmaceutically active particles comprising nicotine (also referred to as "nicotine powder" or "nicotine particles") and optionally particles comprising flavour (also referred to as "flavour particles). The capsule may contain a predetermined amount of nicotine particles and optional flavour particles. The capsule may contain enough nicotine particles to provide at least 2 inhalations or "puffs", or at least about 5 inhalations or "puffs", or at least about 10 inhalations or "puffs". The capsule may contain enough nicotine particles to provide from about 5 to about 50 inhalations or "puffs", or from about 10 to about 30 inhalations or "puffs". Each inhalation or "puff" may deliver from about 0.1 mg to about 3 mg of nicotine particles to the lungs of the user or from about 0.2 mg to about 2 mg of nicotine particles to the lungs of the user or about 1 mg of nicotine particles to the lungs of the user.

The nicotine particles may have any useful concentration of nicotine based on the particular formulation employed. The nicotine particles may have at least about 1 %wt nicotine up to about 30%wt nicotine, or from about 2%wt to about 25%wt nicotine, or from about 3%wt to about 20%wt nicotine, or from about 4%wt to about 15%wt nicotine, or from about 5%wt to about 13%wt nicotine. Preferably, about 50 to about 150 micrograms of nicotine may be delivered to the lungs of the user with each inhalation or "puff".

The capsule may hold or contain at least about 5 mg of nicotine particles or at least about 10 mg of nicotine particles. The capsule may hold or contain less than about 900 mg of nicotine particles, or less than about 300 mg of nicotine particles, or less than 150 mg of nicotine particles. The capsule may hold or contain from about 5 mg to about 300 mg of nicotine particles or from about 10 mg to about 200 mg of nicotine particles.

When flavour particles are blended or combined with the nicotine particles within the capsule, the flavour particles may be present in an amount that provides the desired flavour to each inhalation or "puff" delivered to the user.

The nicotine particles may have any useful size distribution for inhalation delivery preferentially into the lungs of a user. The capsule may include particles other than the nicotine particles. The nicotine particles and the other particles may form a powder system.

The capsule may hold or contain at least about 5 mg of a dry powder (also referred to as a powder system) or at least about 10 mg of a dry powder. The capsule may hold or contain less than about 900 mg of a dry powder, or less than about 300 mg of a dry powder, or less than about 150 mg of a dry powder. The capsule may hold or contain from about 5 mg to about 300 mg of a dry powder, or from about 10 mg to about 200 mg of a dry powder, or from about 25 mg to about 100 mg of a dry powder.

The dry powder or powder system may have at least about 40%, or at least about 60%, or at least about 80%, by weight of the powder system comprised in nicotine particles having a particle size of about 5 micrometres or less, or in a range from about 1 micrometre to about 5 micrometres.

The particles comprising nicotine may have a mass median aerodynamic diameter of about 5 micrometres or less, or in a range from about 0.5 micrometres to about 4 micrometres, or in a range from about 1 micrometres to about 3 micrometres or in a range from about 1.5 micrometres to about 2.5 micrometres. The mass median aerodynamic diameter is preferably measured with a cascade impactor.

The particles comprising flavour may have a mass median aerodynamic diameter of about 20 micrometres or greater, or about 50 micrometres or greater, or in a range from about 50 to about 200 micrometres, or from about 50 to about 150 micrometres. The mass median aerodynamic diameter is preferably measured with a cascade impactor.

The dry powder may have a mean diameter of about 60 micrometres or less, or in a range from about 1 micrometres to about 40 micrometres, or in a range from about 1.5 micrometres to about 25 micrometres. The mean diameter refers to the mean diameter per mass and is preferably measured by laser diffraction, laser diffusion or an electronic microscope.

Nicotine in the powder system or nicotine particles may be a pharmaceutically acceptable free-base nicotine, or nicotine salt or nicotine salt hydrate. Useful nicotine salts or nicotine salt hydrates include nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. The compound combining with nicotine to form the salt or salt hydrate may be chosen based on its expected pharmacological effect.

The nicotine particles preferably include an amino acid. Preferably the amino acid may be leucine such as L-leucine. Providing an amino acid such as L-leucine with the particles comprising nicotine, may reduce adhesion forces of the particles comprising nicotine and may reduce attraction between nicotine particles and thus reduce agglomeration of nicotine particles. Similarly, adhesion forces to particles comprising flavour may also be reduced thus agglomeration of nicotine particles with flavour particles is also reduced. The powder system described herein thus may be a free-flowing material and possess a stable relative particle size of each powder component even when the nicotine particles and the flavour particles are combined.

Preferably, the nicotine may be a surface modified nicotine salt where the nicotine salt particle comprises a coated or composite particle. A preferred coating or composite material may be L-leucine. One particularly useful nicotine particle may be nicotine bitartrate with L-leucine.

The powder system may include a population of flavour particles. The flavour particles may have any useful size distribution for inhalation delivery selectively into the mouth or buccal cavity of a user.

The powder system may have at least about 40%, or at least about 60%, or at least about 80%, by weight of the population of flavour particles of the powder system comprised in particles having a particle size of about 20 micrometres or greater. The powder system may have at least about 40% or at least about 60%, or at least about 80%, by weight of the population of flavour particles of the powder system comprised in particles having a particle size of about 50 micrometres or greater. The powder system may have at least about 40% or at least about 60%, or at least about 80%, by weight of the population of flavour particles of the powder system comprised in particles having a particle size in a range from about 50 micrometre to about 150 micrometres.

The particles comprising flavour may include a compound to reduce adhesion forces or surface energy and resulting agglomeration. The flavour particle may be surface modified with an adhesion reducing compound to form a coated flavour particle. One preferred adhesion reducing compound may be magnesium stearate. Providing an adhesion reducing compound such as magnesium stearate with the flavour particle, especially coating the flavour particle, may reduce adhesion forces of the particles comprising flavour and may reduce attraction between flavour particles and thus reduce agglomeration of flavour particles. Thus, agglomeration of flavour particles with nicotine particles may also be reduced. The powder system described herein thus may possess a stable relative particle size of the particles comprising nicotine and the particles comprising flavour even when the nicotine particles and the flavour particles are combined. The powder system preferably may be free flowing.

Conventional formulations for dry powder inhalation contain carrier particles that serve to increase the fluidization of the active particles since the active particles may be too small to be influenced by simple airflow though the inhaler. The powder system may comprise carrier particles. These carrier particles may be a saccharide such as lactose or mannitol that may have a particle size greater than about 50 micrometres. The carrier particles may be utilized to improve dose uniformity by acting as a diluent or bulking agent in a formulation.

The powder system utilized with the nicotine powder delivery system described herein may be carrier-free or substantially free of a saccharide such as lactose or mannitol. Being carrier-free or substantially free of a saccharide such as lactose or mannitol may allow the nicotine and to be inhaled and delivered to the user's lungs at inhalation or airflow rates that are similar to typical smoking regime inhalation or airflow rates.

The nicotine particles and a flavour may be combined in a single capsule. As described above, the nicotine particles and a flavour may each have reduced adhesion forces that result in a stable particle formulation where the particle size of each component does not substantially change when combined. Alternatively, the powder system includes nicotine particles contained within a single capsule and the flavour particles contained within a second capsule.

The nicotine particles and flavour particles may be combined in any useful relative amount so that the flavour particles are detected by the user when consumed with the nicotine particles. Preferably the nicotine particles and a flavour particles form at least about 90%wt or at least about 95%wt or at least about 99%wt or 100%wt of the total weight of the powder system.

The inhaler and inhaler system may be less complex and have a simplified airflow path as compared to conventional dry powder inhalers. Advantageously, rotation of the capsule within the inhaler body aerosolizes the nicotine particles or powder system and may assist in maintaining a free-flowing powder. Thus, the inhaler article may not require the elevated inhalation rates typically utilized by conventional inhalers to deliver the nicotine particles described above deep into the lungs.

The inhaler article may use a flow rate of less than about 5 L/min or less than about 3 L/min or less than about 2 L/min or about 1.6 L/min. Preferably, the flow rate may be in a range from about 1 L/min to about 3 L/min or from about 1.5 L/min to about 2.5 L/min. Preferably, the inhalation rate or flow rate may be similar to that of Health Canada smoking regime, that is, about 1.6 L/min.

The inhaler system may be used by a consumer like smoking a conventional cigarette or vaping an electronic cigarette. Such smoking or vaping may be characterized by two steps: a first step during which a small volume containing the full amount of nicotine desired by the consumer is drawn into the mouth cavity, followed by a second step during which this small volume comprising the aerosol comprising the desired amount of nicotine is further diluted by fresh air and drawn deeper into the lungs. Both steps are controlled by the consumer. During the first inhalation step the consumer may determine the amount of nicotine to be inhaled. During the second step, the consumer may determine the volume for diluting the first volume to be drawn deeper into the lungs, maximizing the concentration of active agent delivered to the airway epithelial surface. This smoking mechanism is sometimes called "puff-inhale-exhale".

The dry powder utilized with the dry powder inhaler of the invention may eliminate or substantially reduce any exhalation of pharmaceutically active particles during the "exhale" phase. Preferably nearly all, or at least about 99% or at least about 95% or at least 90% of the pharmaceutically active particle has a particle size that is delivered to the lungs but are not small enough to be exhaled by tidal breathing. This pharmaceutically active particle size may be in a range from about 0.75 micrometres to about 5 micrometres, or from 0.8 micrometres to about 3 micrometres, or from 0.8 micrometres to about 2 micrometres.

The holder for an inhaler article may be combined with an inhaler article (described herein) containing a capsule for activating the inhaler article by piercing the capsule, providing reliable activation of the capsule (by puncturing the capsule with the piercing element of the holder) within inhaler article, and releasing the particles contained inside the capsule and enabling the article to deliver the particles to a consumer. The holder is separate from the inhaler article, but the consumer may utilize both the inhaler article and the holder while consuming the particles released within the inhaler article. A plurality of these inhaler articles may be combined with a holder to form a system or kit. A single holder may be utilized on 10 or more, or 25 or more, or 50 or more, or 100 or more, inhaler articles to activate (puncture or pierce) a capsule contained within each inhaler article and provide reliable activation and optionally, a visual indication (marking), for each inhaler article of the activation of the inhaler article.

A holder for an inhaler article includes a housing comprising a housing cavity for receiving an inhaler article and a sleeve configured to retain an inhaler article within the housing cavity. The sleeve comprising a sleeve cavity and being movable within the housing cavity along the longitudinal axis of the housing. The sleeve comprises a first open end and a second opposing end. The first open end is configured to receive the distal end of the inhaler article. The second opposing end of the sleeve is configured to contact the distal end of the inhaler article. The sleeve second opposing end is configured to direct substantially all inhalation air to flow through the inhaler article at least one air inlet extending in a direction that is tangential to the central passage.

An inhaler system includes the inhaler article described herein with a capsule disposed within the capsule cavity and a holder receiving the inhaler article. The holder includes a housing having a housing cavity for receiving an inhaler article and a sleeve configured to retain an inhaler article within the housing cavity. The sleeve includes a sleeve cavity and the sleeve is movable within the housing cavity along a longitudinal axis of the housing. The sleeve includes a first open end and a second opposing end. The first open end is configured to receive an inhaler article and the second opposing end of the sleeve is configured contact the distal end of the inhaler article.

The holder may further comprise a piercing element fixed to and extending from a housing inner surface. The piercing element may be configured to extend through the second opposing end of the sleeve and into the capsule cavity to pierce the capsule along a longitudinal axis of the housing.

The holder may further include a spring element configured to bias the sleeve toward the open proximal end of the housing, and between a relaxed and compressed position. The spring element may be contained within the inhaler article cavity of the holder and be compressed as the movable sleeve and inhaler article move toward the piercing element. The spring element may be between the sleeve and distal end of the housing and contact the sleeve and distal end of the housing. The spring element may be between the distal end of the sleeve and the distal end of the housing. The spring element may contact the distal end of the sleeve and the distal end of the housing. The spring element may be disposed about the piercing element. The spring element may be co-axial with the piercing element. The spring element may be a conical spring.

The spring element biases the inhaler article away from the piercing element. In use, a user may insert an inhaler article into the inhaler article cavity of the holder. By doing this, the spring may be compressed allowing the inhaler article to move towards the distal end of the inhaler article cavity. Eventually, the piercing element may penetrate a capsule disposed within the inhaler article. Once this happens, the user may release the inhaler article, allowing the spring to bias the inhaler article towards the proximal end of the inhaler article cavity and away from the piercing element. The user may then inhale on the proximal end of the inhaler article.

The sleeve may define a first air inlet zone comprising at least one air aperture through the sleeve. The first air inlet zone is proximate to a proximal end of the sleeve. The first air inlet zone is configured to allow air to flow from an inside of the sleeve to an airflow channel formed between the sleeve and the housing inner surface. The sleeve may comprise a second air inlet zone comprising at least one air aperture through the sleeve. The second air inlet zone is proximate to a distal end of the sleeve. The second air inlet zone is configured to allow air to flow from the airflow channel to an inside of the sleeve.

All scientific and technical terms used herein have meanings commonly used in the art unless otherwise specified. The definitions provided herein are to facilitate understanding of certain terms used frequently herein.

As used herein, the singular forms "a", "an", and "the" encompass embodiments having plural referents, unless the content clearly dictates otherwise.

As used herein, "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open-ended sense, and generally mean "including, but not limited to". It will be understood that "consisting essentially of", "consisting of", and the like are subsumed in "comprising," and the like.

The words "preferred" and "preferably" refer to embodiments of the technology that may afford certain benefits, under certain circumstances. However, other embodiments may also be preferred, under the same or other circumstances. The invention is defined by the appended claims.

The technology will now be further described with reference to the figures in which:
FIG. 1 is a cross-sectional diagram of an illustrative inhaler article along a longitudinal axis;
FIG. 2 is a cross-sectional diagram of another illustrative inhaler article along a longitudinal axis;
FIG.s 3-6 are cross-sectional schematic diagrams of illustrative tubular elements with one to four tangential air inlets;

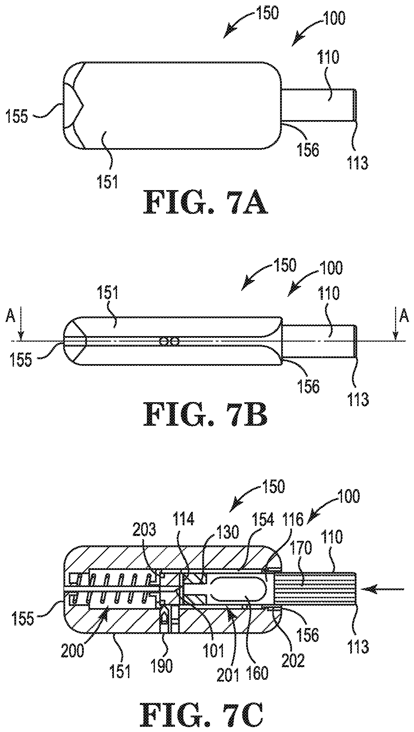

FIG. 7A is a side elevation schematic diagram of an illustrative flat holder useful with an inhaler article;
FIG. 7B is a top elevation schematic diagram of the illustrative flat holder of FIG. 7A; and
FIG. 7C is a cross-sectional schematic diagram of the illustrative flat holder of FIG. 7B taken along line A-A.

The schematic drawings are not necessarily to scale and are presented for purposes of illustration and not limitation. The drawings depict one or more aspects described in this disclosure. However, it will be understood that other aspects not depicted in the drawing fall within the scope of this disclosure.

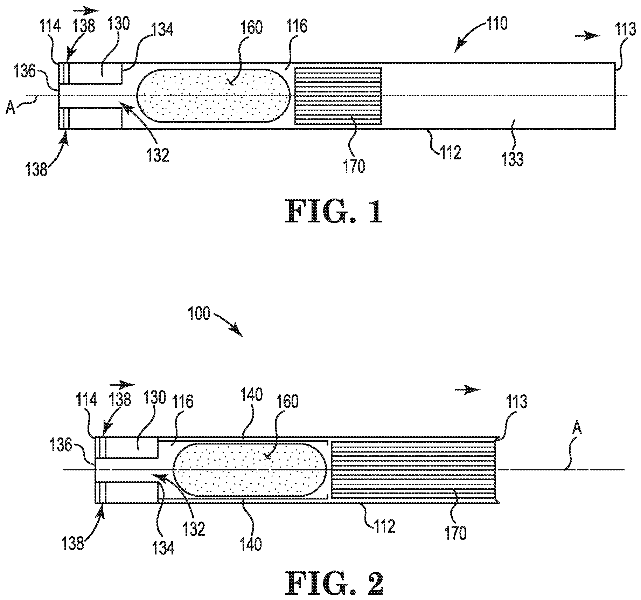

FIGS. 1 and 2 illustrate exemplary inhaler articles 110. The inhaler article 110 may include a capsule 160 disposed within inhaler article 110.

The inhaler article 110 includes a body 112 extending along a longitudinal axis "A" from a mouthpiece end 113 to a distal end 114. The mouthpiece end 113 forms a downstream end and distal end 114 forms an upstream end of the inhaler article 110. In other words, the mouthpiece end 113 is downstream from the distal end, as indicated with arrows. A capsule cavity 116 is defined within the body 112. A tubular element 130 extends from the distal end 114 towards the capsule cavity 116.

The tubular element 130 defines a central passage 132 extending between a first end 134 and a second opposing end 136 defining a distal end surface 136 of the inhaler article 110, and the first end 134 facing the sleeve cavity 116. The tubular element 130 comprising at least one air inlet 138 allowing air to enter into the central passage 132. The at least one air inlet 138 extending in a direction that is tangential to the central passage 132.

In this example, the second opposing end 136 is shown as being located directly at the distal end 114 of the body 112. Furthermore, in this example there is no component between the second opposing end 136 and the distal end 114 of the body 112. This simplifies the construction of the inhaler article 110, and facilities air flow through the inhaler article 110. However, it should be appreciated that the tubular element 130 being "at" the distal end 114 should be interpreted as the tubular element 130 being around the same location as the distal end 114. For instance, the tubular element 130 may be offset from the distal end 114 along the longitudinal axis either towards or away from the mouthpiece end 113

In this example, the second opposing end 136 is shown as being located directly at the distal end 114 of the body 112. However, it should be appreciated that the tubular element 130 being at the distal end 114 should be interpreted as the tubular element 130 being around the same location as the distal end 114. For instance, the tubular element 130 may be offset from the distal end 114 along the longitudinal axis either towards or away from the mouthpiece end 113.

The tubular element 130, the capsule cavity 116 (and capsule 160 if present), and a porous support element 170 may be axially aligned and serially disposed within the body 112. The tubular element 130 may form an upstream or distal end or boundary of the capsule cavity 116. The capsule cavity 116 may define a space configured to contain the capsule 160. The capsule cavity 116 may have a fixed cavity length bounded on the first end 134 of the tubular element 130 and bounded on the downstream end by the porous support element 170. As illustrated in FIG. 1, the porous support element 170 may be disposed within a mouthpiece air channel portion 133 of the body 110. The mouthpiece air channel portion 133 may extend beyond the porous support element 170 to the mouthpiece end 113.

The tubular element 130 may be disposed within the distal end 114 of the body 112. The second opposing end 136 of the tubular element 130 may be substantially aligned with the distal end 114 of the body 112.

As illustrated in FIG. 2, the inhaler article 110 may include an insert body 140 defining the capsule cavity 116 to contain the capsule 160 and provide support and rigidity to the body 112 along the capsule cavity 116. The filter or porous support element 170 extends from the capsule cavity 116 to the mouthpiece end 113.

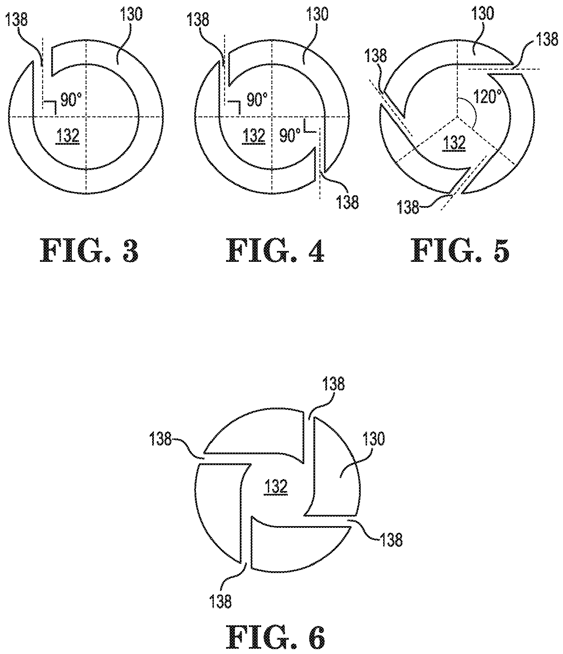

FIG.s 3-6 are cross-sectional schematic diagrams of illustrative tubular elements 130 with central passageways 132 with one to four tangential air inlets 138. The inner diameter of the tubular elements 130 may be about 4 mm. FIG. 3 illustrates a single tangential air inlet 138 having a diameter of about 1.45 mm. FIG. 4 illustrates two tangential air inlets 138 each having a diameter of about 1 mm and entering the tubular element 130 at 180 degrees from each other. FIG. 5 illustrates three tangential air inlets 138 each having a diameter of about 0.85 mm entering the tubular element 130 at 120 degrees from each other. FIG. 6 illustrates four tangential air inlets 138 each having a diameter of about 0.6 mm entering the tubular element 130 at 90 degrees from each other.

With reference to FIG.s 7A-7C, an inhaler system 100 includes the inhaler article 110 and a holder 150. The inhaler article 110 comprises the body 112 defining an inhaler outer surface. The inhaler article 110 may be similar to either exemplary inhaler article 110 of FIG. 1 or FIG. 2. The body 112 extends along an inhaler longitudinal axis from mouthpiece, or proximal end 113 to the distal end 114 a body length.

The holder 150 for the inhaler article 110 includes, a housing 151 and a piercing element 101. The holder 150 may also include a marking element 190. The housing 151 has a housing outer surface and a housing inner surface. The housing inner surface defines an inhaler article cavity 154 configured to receive the inhaler article 110. The housing 151 extends along a housing longitudinal axis from a distal end 155 to an open proximal end 156 defining a housing length. The open proximal end 156 is configured to receive the distal end 114 of an inhaler article 110 into the inhaler article cavity 154.

The holder 150 for an inhaler article 110 includes the housing 151 comprises the housing cavity 154 for receiving an inhaler article 110 and a sleeve 201 configured to retain the inhaler article 110 within the housing cavity 154. The sleeve 201 comprises a sleeve cavity and being movable within the housing cavity 154 along the longitudinal axis of the housing. The sleeve 201 comprises a first open end 202 and a second opposing end 203. The first open end 202 is configured to receive the distal end 114 of the inhaler article 110. The second opposing end 203 of the sleeve 201 is configured to contact the distal end 114 of the inhaler article 110. The sleeve second opposing end 203 is configured to direct substantially all inhalation air to flow through the inhaler article 110 at least one air inlet extending in a direction that is tangential to the central passage.

The piercing element 101 is fixed to and extends from the housing inner surface, into the inhaler article cavity 154 along a piercing element longitudinal axis a piercing element length. The piercing element 101 is recessed from the open proximal end 156 a recessed distance. The piercing element 101 is configured to be movable to extend through the central passage of the tubular element 130 and to the capsule 160 housed within the capsule cavity 116 to pierce the capsule 160. A spring element 200 may bias the inhaler article 110 away from the piercing element 101.

The exemplary embodiments described above are not limiting. Other embodiments consistent with the exemplary embodiments described above will be apparent to those skilled in the art.

## Claims

1. An inhaler article (110) comprising:
a body (112) extending along a longitudinal axis from a mouthpiece end (113) to a distal end (114);
a capsule cavity (116) defined within the body (112);
a tubular element (130) extending from the distal end (114) to the capsule cavity (116), the tubular element (130) defining a central passage (132) having a uniform open diameter extending from the distal end (114) to the capsule cavity (116), the tubular element (130) comprising at least one tangential air inlet (138) for allowing air to enter into the central passage (132), the at least one tangential air inlet (138) extending in a direction that is tangential to the central passage (132).

2. The inhaler article (110) according to claim 1, wherein the at least one tangential air inlet (138) is proximate to the distal end (114) of the inhaler article body (112).

3. The inhaler article (110) according to claim 1 or 2, wherein the central passage (132) comprises a first end (134) defining an upstream boundary of the capsule cavity (116) and a second opposing end (136) defining the distal end (114) of the inhaler article body (112).

4. The inhaler article (110) according to claim 3, wherein the second opposing end (136) defines an open distal end (114) of the of the inhaler article body (112).

5. The inhaler article (110) according to any preceding claim, wherein central passage (132) extends along the longitudinal axis of the body (112) of the inhaler article (110) and defines an opening at the distal end (114) of the body (112) of the inhaler article (110) that is coaxial with the longitudinal axis of the body (112) of the inhaler article (110).

6. The inhaler article (110) according to any preceding claim, wherein a lateral distance between the at least one tangential air inlet (138) and the capsule cavity (116) is greater than a lateral distance from the distal end (114) of the inhaler article body (112) to the at least one tangential air inlet (138).

7. The inhaler article (110) according to any preceding claim, wherein the tubular element (130) comprises at least two air inlets (138), the at least two air inlets (138) extend in a direction that is tangential to the central passage (132).

8. The inhaler article (110) according to any preceding claim, wherein the tubular element (130) comprises at least three air inlets (138), the at least three air inlets (138) extend in a direction that is tangential to the central passage (132).

9. The inhaler article (110) according to any preceding claim, wherein the tubular element (130) is formed of a cellulose acetate material or a polylactic acid material.

10. The inhaler article (110) according to any preceding claim, further comprising a capsule (160) retained within the capsule cavity (116).

11. The inhaler article (110) according to claim 10, wherein the tubular element (130) central passage (132) has a diameter in a range from about 50% to about 90% of a diameter of the capsule (160).

12. An inhaler system (100) comprising:
the inhaler article (110) according to any preceding claim, and a capsule (160) disposed within the capsule cavity (116); and
a holder (150) receiving the inhaler article (110), the holder (150) comprising:
a housing (151) comprising a housing cavity (154) for receiving an inhaler article (110); and
a sleeve (201) configured to retain an inhaler article (110) within the housing cavity (154), the sleeve (201) comprising a sleeve cavity and being movable within the housing cavity (154) along a longitudinal axis of the housing (151), wherein the sleeve (201) comprises a first open end (202) and a second opposing end (203), the first open end (202) is configured to receive an inhaler article (110) and the second opposing end (203) of the sleeve (201) is configured contact the distal end (114) of the inhaler article (110).

13. The inhaler system (100) according to claim 12, wherein the sleeve second opposing end (203) is configured to direct substantially all inhalation air to flow through the the inhaler article (110) at least one tangential air inlet (138) extending in a direction that is tangential to the central passage (132).

14. The inhaler system (100) according to claim 12 or 13, further comprising a piercing element (101) fixed to and extending from a housing inner surface, the piercing element (101) being configured to extend through the second opposing end (203) of the sleeve (201) and into the capsule cavity (116) to pierce the capsule (160) along a longitudinal axis of the housing (151).

15. The inhaler system (100) according to one of claims 12 to 14, wherein the capsule (160) contains pharmaceutically active particles comprising nicotine, the pharmaceutically active particles having a mass median aerodynamic diameter of about 5 micrometres or less, or in a range from about 0.5 micrometres to about 4 micrometres, or in a range from about 1 micrometres to about 3 micrometres.

## Patentansprüche

1. Inhalatorartikel (110), umfassend:
einen Körper (112), der sich entlang einer Längsachse von einem Mundstückende (113) zu einem distalen Ende (114) erstreckt;
einen innerhalb des Körpers (112) definierten Kapselhohlraum (116) ;
ein rohrförmiges Element (130), das sich von dem distalen Ende (114) zu dem Kapselhohlraum (116) erstreckt, wobei das rohrförmige Element (130) einen zentralen Durchgang (132) mit einem gleichmäßig offenen Durchmesser definiert, der sich von dem distalen Ende (114) zu dem Kapselhohlraum (116) erstreckt, wobei das rohrförmige Element (130) wenigstens einen tangentialen Lufteinlass (138) zum Ermöglichen des Eintretens von Luft in den zentralen Durchgang (132) aufweist, wobei sich der wenigstens eine tangentiale Lufteinlass (138) in einer Richtung erstreckt, die tangential zu dem zentralen Durchgang (132) ist.

2. Inhalatorartikel (110) nach Anspruch 1, wobei der wenigstens eine tangentiale Lufteinlass (138) in der Nähe des distalen Endes (114) des Inhalatorartikelkörpers (112) liegt.

3. Inhalatorartikel (110) nach Anspruch 1 oder 2, wobei der zentrale Durchgang (132) ein erstes Ende (134), das eine vorgelagerte Begrenzung des Kapselhohlraums (116) umfasst, und ein zweites, gegenüberliegendes Ende (136) aufweist, das das distale Ende (114) des Inhalatorartikelkörpers (112) definiert.

4. Inhalatorartikel (110) nach Anspruch 3, wobei das zweite gegenüberliegende Ende (136) ein offenes distales Ende (114) des Inhalatorartikelkörpers (112) definiert.

5. Inhalatorartikel (110) nach einem beliebigen vorhergehenden Anspruch, wobei sich der zentrale Durchgang (132) entlang der Längsachse des Körpers (112) des Inhalatorartikels (110) erstreckt und eine Öffnung an dem distalen Ende (114) des Körpers (112) des Inhalatorartikels (110) definiert, die koaxial mit der Längsachse des Körpers (112) des Inhalatorartikels (110) ist.

6. Inhalatorartikel (110) nach einem beliebigen vorhergehenden Anspruch, wobei ein seitlicher Abstand zwischen dem wenigstens einen tangentialen Lufteinlass (138) und dem Kapselhohlraum (116) größer ist als ein seitlicher Abstand von dem distalen Ende (114) des Inhalatorartikelkörpers (112) zu dem wenigstens einen tangentialen Lufteinlass (138).

7. Inhalatorartikel (110) nach einem beliebigen vorhergehenden Anspruch, wobei das rohrförmige Element (130) wenigstens zwei Lufteinlässe (138) aufweist, wobei sich die wenigstens zwei Lufteinlässe (138) in eine tangential zu dem zentralen Durchgang (132) verlaufende Richtung erstrecken.

8. Inhalatorartikel (110) nach einem beliebigen vorhergehenden Anspruch, wobei das rohrförmige Element (130) wenigstens drei Lufteinlässe (138) aufweist, wobei sich die wenigstens drei Lufteinlässe (138) in eine tangential zu dem zentralen Durchgang (132) verlaufende Richtung erstrecken.

9. Inhalatorartikel (110) nach einem beliebigen vorhergehenden Anspruch, wobei das rohrförmige Element (130) aus einem Celluloseacetatmaterial oder einem Polymilchsäurematerial gebildet ist.

10. Inhalatorartikel (110) nach einem beliebigen vorhergehenden Anspruch, ferner umfassend eine innerhalb des Kapselhohlraums (116) gehaltene Kapsel (160).

11. Inhalatorartikel (110) nach Anspruch 10, wobei der zentrale Durchgang (132) des rohrförmigen Elements (130) einen Durchmesser in einem Bereich von etwa 50 % bis etwa 90 % des Durchmessers der Kapsel (160) aufweist.

12. Inhalatorsystem (100), umfassend:
den Inhalatorartikel (110) nach einem beliebigen vorhergehenden Anspruch und eine innerhalb des Kapselhohlraums (116) angeordnete Kapsel (160); und
einen Halter (150) zum Aufnehmen des Inhalatorartikels (110), der Halter (150) umfassend:
ein Gehäuse (151), umfassend einen Gehäusehohlraum (154) zum Aufnehmen eines Inhalatorartikels (110); und
eine zum Halten eines Inhalatorartikels (110) innerhalb des Gehäusehohlraums (154) ausgelegte Hülse (201), wobei die Hülse (201) einen Hülsenhohlraum umfasst und innerhalb des Gehäusehohlraums (154) entlang einer Längsachse des Gehäuses (151) beweglich ist, wobei die Hülse (201) ein erstes offenes Ende (202) und ein zweites gegenüberliegendes Ende (203) aufweist, wobei das erste offene Ende (202) zum Aufnehmen eines Inhalatorartikels (110) ausgelegt ist und das zweite gegenüberliegende Ende (203) der Hülse (201) zum Kontaktieren des distalen Endes (114) des Inhalatorartikels (110) ausgelegt ist.

13. Inhalatorsystem (100) nach Anspruch 12, wobei das zweite gegenüberliegende Ende (203) der Hülse zum Leiten im Wesentlichen der gesamten Inhalationsluft durch den Inhalatorartikel (110) ausgelegt ist, wobei wenigstens ein tangentialer Lufteinlass (138) sich in eine Richtung erstreckt, die tangential zu dem zentralen Durchgang (132) ist.

14. Inhalatorsystem (100) nach Anspruch 12 oder 13, ferner umfassend ein an einer Gehäuseinnenfläche befestigtes und sich von dieser erstreckendes Durchstechelement (101), wobei das Durchstechelement (101) zum sich Erstrecken durch das zweite gegenüberliegende Ende (203) der Hülse (201) und in den Kapselhohlraum (116) zum Durchstechen der Kapsel (160) entlang einer Längsachse des Gehäuses (151) ausgelegt ist.

15. Inhalatorsystem (100) nach einem der Ansprüche 12 bis 14, wobei die Kapsel (160) pharmazeutisch aktive Partikel enthält, die Nikotin umfassen, wobei die pharmazeutisch wirksamen Partikel einen mittleren aerodynamischen Massendurchmesser von etwa 5 Mikrometer oder weniger oder in einem Bereich von etwa 0,5 Mikrometer bis etwa 4 Mikrometer oder in einem Bereich von etwa 1 Mikrometer bis etwa 3 Mikrometer aufweisen.

## Revendications

1. Article inhalateur (110) comprenant :
un corps (112) s'étendant le long d'un axe longitudinal depuis une extrémité d'embout buccal (113) jusqu'à une extrémité distale (114) ;
une cavité de capsule (116) définie au sein du corps (112) ;
un élément tubulaire (130) s'étendant depuis l'extrémité distale (114) jusqu'à la cavité de capsule (116), l'élément tubulaire (130) définissant un passage central (132) ayant un diamètre ouvert uniforme s'étendant depuis l'extrémité distale (114) jusqu'à la cavité de capsule (116), l'élément tubulaire (130) comprenant au moins une entrée d'air tangentielle (138) pour permettre à l'air d'entrer dans le passage central (132), l'au moins une entrée d'air tangentielle (138) s'étendant dans une direction qui est tangentielle au passage central (132).

2. Article inhalateur (110) selon la revendication 1, dans lequel l'au moins une entrée d'air tangentielle (138) est à proximité de l'extrémité distale (114) du corps d'article inhalateur (112).

3. Article inhalateur (110) selon la revendication 1 ou 2, dans lequel le passage central (132) comprend une première extrémité (134) définissant une limite amont de la cavité de capsule (116) et une deuxième extrémité opposée (136) définissant l'extrémité distale (114) du corps d'article inhalateur (112).

4. Article inhalateur (110) selon la revendication 3, dans lequel la deuxième extrémité opposée (136) définit une extrémité distale ouverte (114) du corps d'article inhalateur (112).

5. Article inhalateur (110) selon l'une quelconque des revendications précédentes, dans lequel le passage central (132) s'étend le long de l'axe longitudinal du corps (112) de l'article inhalateur (110) et définit une ouverture au niveau de l'extrémité distale (114) du corps (112) de l'article inhalateur (110) qui est coaxiale à l'axe longitudinal du corps (112) de l'article inhalateur (110).

6. Article inhalateur (110) selon l'une quelconque des revendications précédentes, dans lequel une distance latérale entre l'au moins une entrée d'air tangentielle (138) et la cavité de capsule (116) est supérieure à une distance latérale depuis l'extrémité distale (114) du corps d'article inhalateur (112) jusqu'à l'au moins une entrée d'air tangentielle (138).

7. Article inhalateur (110) selon l'une quelconque des revendications précédentes, dans lequel l'élément tubulaire (130) comprend au moins deux entrées d'air (138), les au moins deux entrées d'air (138) s'étendant dans une direction qui est tangentielle au passage central (132).

8. Article inhalateur (110) selon l'une quelconque des revendications précédentes, dans lequel l'élément tubulaire (130) comprend au moins trois entrées d'air (138), les au moins trois entrées d'air (138) s'étendant dans une direction qui est tangentielle au passage central (132).

9. Article inhalateur (110) selon l'une quelconque des revendications précédentes, dans lequel l'élément tubulaire (130) est formé d'une matière d'acétate de cellulose ou d'une matière d'acide polylactique.

10. Article inhalateur (110) selon l'une quelconque des revendications précédentes, comprenant en outre une capsule (160) retenue au sein de la cavité de capsule (116).

11. Article inhalateur (110) selon la revendication 10, dans lequel le passage central (132) de l'élément tubulaire (130) a un diamètre dans une plage d'environ 50 % à environ 90 % d'un diamètre de la capsule (160).

12. Système inhalateur (100) comprenant :
l'article inhalateur (110) selon l'une quelconque des revendications précédentes, et une capsule (160) disposée au sein de la cavité de capsule (116) ; et
un support (150) recevant l'article inhalateur (110), le support (150) comprenant :
un logement (151) comprenant une cavité de logement (154) destinée à recevoir un article inhalateur (110) ; et
un manchon (201) configuré pour retenir un article inhalateur (110) au sein de la cavité de logement (154), le manchon (201) comprenant une cavité de manchon et étant mobile au sein de la cavité de logement (154) le long d'un axe longitudinal du logement (151), dans lequel le manchon (201) comprend une première extrémité ouverte (202) et une deuxième extrémité opposée (203), la première extrémité ouverte (202) est configurée pour recevoir un article inhalateur (110) et la deuxième extrémité opposée (203) du manchon (201) est configurée pour être en contact avec l'extrémité distale (114) de l'article inhalateur (110).

13. Système inhalateur (100) selon la revendication 12, dans lequel la deuxième extrémité opposée (203) du manchon est configurée pour diriger sensiblement tout l'air d'inhalation pour qu'il s'écoule à travers l'article inhalateur (110), au moins une entrée d'air tangentielle (138) s'étendant dans une direction qui est tangentielle au passage central (132).

14. Système inhalateur (100) selon la revendication 12 ou 13, comprenant en outre un élément de perçage (101) attaché à une surface intérieure de logement et s'étendant depuis celle-ci, l'élément de perçage (101) étant configuré pour s'étendre à travers la deuxième extrémité opposée (203) du manchon (201) et dans la cavité de capsule (116) pour percer la capsule (160) le long d'un axe longitudinal du logement (151).

15. Système inhalateur (100) selon l'une des revendications 12 à 14, dans lequel une capsule (160) contient des particules pharmaceutiquement actives comprenant de la nicotine, les particules pharmaceutiquement actives ayant un diamètre aérodynamique moyen en masse d'environ 5 micromètres ou moins, ou dans une plage d'environ 0,5 micromètre à environ 4 micromètres, ou dans une plage d'environ 1 micromètre à environ 3 micromètres.
